(19) Europäisches Patentamt
European Patent Office
Office européen des brevets

(11) Veröffentlichungsnummer: **0 553 385 B1**

(12) **EUROPÄISCHE PATENTSCHRIFT**

(45) Veröffentlichungstag der Patentschrift: **29.03.95**

(51) Int. Cl.[6]: **C07C 229/22**, A61K 31/615

(21) Anmeldenummer: **92101618.4**

(22) Anmeldetag: **31.01.92**

# (54) Salicyloyl-carnitin und Verfahren zu seiner Herstellung.

(43) Veröffentlichungstag der Anmeldung:
**04.08.93 Patentblatt 93/31**

(45) Bekanntmachung des Hinweises auf die Patenterteilung:
**29.03.95 Patentblatt 95/13**

(84) Benannte Vertragsstaaten:
**AT BE DE ES FR GB IT LU NL PT SE**

(56) Entgegenhaltungen:
**CH-A- 679 395**
**FR-A- 2 429 776**

**ORGANIC MASS SPECTROMETRY Bd. 3, Nr. 11, 1970, Seiten 1433 - 1438; G.HVISTENDAHL ET AL: 'Mass spectrometry of onium compounds - II Carnitine and O-acyl derivatives'**

**'THE MERCK INDEX, 10. Auflage' 1983 , MERCK & CO INC , RAHWAY, NJ, US**

(73) Patentinhaber: **LONZA AG**
**(Geschäftsleitung: 4002 Basel)**
**CH-3945 Gampel/Wallis (CH)**

(72) Erfinder: **Meul, Thomas, Dr.**
**Meisenweg 1**
**Visp (Kanton Wallis) (CH)**
Erfinder: **Deshusses, Jacques, Dr.**
**Route A. Ferraud 87**
**Bernex (Kanton Genf) (CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P.**
**Barz**
**Siegfriedstrasse 8**
**D-80803 München (DE)**

EP 0 553 385 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 3-(2-Hydroxybenzoyloxy)-4-(trimethylammonio)-buttersäurebetain der allgemeinen Formel

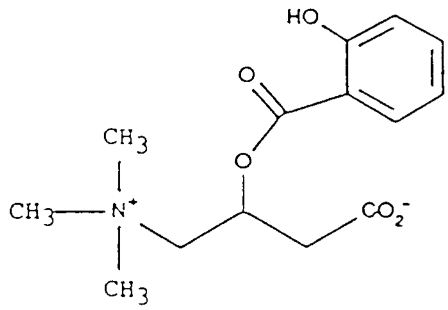

I

in racemischer und optisch aktiver Form, dessen pharmazeutisch akzeptable Salze sowie ein Verfahren zu dessen Herstellung. 3-(2-Hydroxybenzoyloxy)-4-(trimethylammonio)-buttersäurebetain ist als Ester der Salicylsäure mit Carnitin (Salicyloylcarnitin) ein Salicylsäurederivat mit vielversprechenden therapeutische Eigenschaften.

Salicylsäure wird in Form ihres Acetylderivats in grossem Umfang als Analgetikum eingesetzt. Obwohl dieses Acetylderivat (bekannt u.a. als Aspirin$^{R}$) ursprünglich entwickelt wurde, um störende Nebenwirkungen der schon früher bekannten Salicylsäure zu verringern, ist es dennoch mit einigen Eigenschaften behaftet, die seine Anwendungsmöglichkeiten einschränken. Zu diesen ungünstigen Eigenschaften zählt vor allem seine geringe Wasserlöslichkeit, insbesondere in saurem Milieu, also beispielsweise im Magensaft. Die geringe Löslichkeit kann bei oraler Verabreichung von wässrigen Lösungen zum Ausfallen des Wirkstoffs im Magen führen. Dieser Effekt ist nicht nur bei Personen mit empfindlicher oder vorgeschädigter Magenschleimhaut unerwünscht, weil er bei diesen Personen zu ernsthaften Nebenwirkungen führen kann, sondern er verzögert ganz allgemein die Resorption und damit auch den Eintritt der analgetischen Wirkung. Ausserdem kann Acetylsalicylsäure praktisch nur oral, aber nicht parenteral, also beispielsweise intravenös oder intraperitoneal, oder topisch appliziert werden. Gerade wegen des schnellen Wirkungseintritts und/oder der Schonung des Gastrointestinaltrakts wäre aber eine parenterale Applikation oft wünschenswert.

Aufgabe der vorliegenden Erfindung war daher, ein Salicylsäurederivat zur Verfügung zu stellen, das auch im sauren Bereich gut wasserlöslich ist, leicht resorbiert wird, eine möglichst geringe Toxizität aufweist und sowohl enteral als auch parenteral oder topisch appliziert werden kann und in allen Darreichungsformen eine rasch einsetzende analgetische Wirkung zeigt.

Erfindungsgemäss wird diese Aufgabe durch das 3-(2-Hydroxybenzoyloxy)-4-(trimethylammonio)-buttersäurebetain nach Patentanspruch 1 gelöst. Die Verbindung weist ein asymmetrisches Kohlenstoffatom auf und kann daher in zwei spiegelbildlichen optisch aktiven Formen und als racemisches Gemisch auftreten. Bevorzugt ist dabei das Enantiomer mit (R)-Konfiguration, das sich von dem natürlich vorkommenden L-Carnitin ableitet.

Die günstigen physikalisch-chemischen Eigenschaften, wie hohe Wasserlöslichkeit und günstiger pH-Wert der Lösung, werden allerdings auch von dem (S)-Enantiomer und dem Racemat erreicht.

Untersuchungen an Ratten zeigten bereits eine äusserst geringe akute Toxizität von Salicyloyl-L-carnitin. Dosen bis zu 1000 mg/kg Körpergewicht wurden oral ohne weiteres vertragen und bei intravenöser und intraperitonealer Verabreichung therapeutischer Mengen wurden keine schädlichen Nebenwirkungen beobachtet.

In einer ersten Studie wurde die analgetische Wirkung von Saliyloyl-L-Carnitin mit der von Aspirin nach oraler, intraperitonealer und intravenöser Applikation an Ratten verglichen.

Bei oraler Applikation zeigte Salicyloyl-L-Carnitin eine gegenüber Acetylsalicylsäure um 1,5 h retardierte Wirkung, bei intraperitonealer und intravenäser Applikation eine bereits nach 10 bis 15 min einsetzende deutliche analgetische Wirkung.

Selbstverständlich liegt es ebenfalls im Rahmen der Erfindung, Salze des Salicyloylcarnitins mit pharmazeutisch akzeptablen Säuren zu bilden und die Verbindung in dieser Form zu verwenden.

Das Salicyloylcarnitin wird erfindungsgemäss dadurch hergestellt, dass ein Carnitin-Hydrohalogenid, vorzugsweise das Hydrochlorid, zunächst mit einem 2-Methoxybenzoylhalogenid (o-Anisoylhalogenid), vorzugsweise dem Säurechlorid, zu dem entsprechenden 3-(2-Methoxybenzoyloxy)-4-(trimethylammonio)-buttersäurebetain-Hydrohalogenid (o-Anisoylcarnitin-Hydrohalogenid) verestert wird.

Als Lösungsmittel wird zweckmässig ein polares protisches Lösungsmittel verwendet, das gegen Chlorwasserstoff inert ist. Vorteilhaft werden hierzu niedrige aliphatische Carbonsäuren, wie Essigsäure oder Ameisensäure, eingesetzt, besonders bevorzugt ist Trichloressigsäure. Die Reaktionstemperatur in diesem Lösungsmittel beträgt vorteilhaft 50 bis 90 °C, die Reaktionsdauer 1 bis 4 h.

Durch anschliessende Behandlung mit einem Ueberschuss von Bromwasserstoff in Eisessig gelingt es, die Methoxygruppe in eine Hydroxygruppe zu überführen. Die Umsetzung mit Bromwasserstoff wird zweckmässig bei 20 bis 80 °C durchgeführt. Durch den Bromwasserstoff-Ueberschuss wird dabei die Zielverbindung in Form des Hydrobromids erhalten, das in kristalliner Form isoliert werden kann. Für die Verwendung zu pharmazeutischen Zwecken wird das Hydrobromid zweckmässig mit einer Base in das freie Betain übergeführt. Aus Löslichkeitsgründen wird dies vorteilhaft durch Behandeln mit einem schwach basischen Anionenaustauscherharz erreicht. Bei der grundsätzlich ebenfalls möglichen Verwendung einer gelösten Base würde nämlich ein Salz entstehen, das ähnliches Löslichkeitsverhalten wie das Betain zeigt und deshalb nicht leicht abzutrennen wäre. Ein schwach basischer Anionenaustauscher, der als funktionelle Gruppen primäre, sekundäre oder tertiäre Aminogruppen trägt, hat zudem den Vorteil, dass er weder die Estergruppe des Produkts hydrolysiert, noch dieses über die schwach saure Phenolfunktion bindet.

Das beschriebene Verfahren eignet sich selbstverständlich, je nach Ausgangsmaterial, gleichermassen für die Herstellung von racemischem wie von optisch aktivem Salicyloylcarnitin.

Ebenso liegt es im Rahmen des erfindungsgemässen Verfahrens, anschliessend das Betain durch Zugabe einer pharmazeutisch akzeptablen Säure in ein entsprechendes Salz überzuführen.

Die nachfolgenden Beispiele verdeutlichen die Durchführung des erfindungsgemässen Herstellungsverfahrens.

## Beispiel 1

(R)-(-)-3-(2-Methoxybenzoyloxy)-4-(trimethylammonio)-buttersäurebetain-Hydrochlorid (o-Anisoyl-L-carnitin-Hydrochlorid)

In 152,0 g Trichloressigsäure wurden bei 80 °C 61,8 g L-Carnitin-Hydrochlorid gelöst. Innerhalb von 30 min wurden bei 80 °C 80,0 g 2-Methoxybenzoylchlorid zugetropft. Das Reaktionsgemisch wurde noch 90 min bei derselben Temperatur gerührt, auf 30 °C abgekühlt und unter Rühren mit 500 ml Diethylether und 200 ml Ethylacetat versetzt. Das Gemisch wurde 30 min zum Rückfluss erhitzt, wobei das Produkt auskristallisierte.
Das Rohprodukt wurde abfiltriert und getrocknet (Rohausbeute: 112,6 g), anschliessend in 200 ml Isopropanol bei 80 °C aufgeschlämmt, filtriert und zweimal mit je 50 ml Isopropanol auf dem Filter gewaschen.
Ausbeute: 75,7 g (73,0%) farblose Kristalle
Schmp.: 186-190 °C
$[\alpha]_D^{20}$ = -28,8 ° (c = 1, Wasser)

| | |
|---|---|
| $^1$H-NMR (DMSO-$d_6$, 300 MHz) δ | 7,02-7,75 (m, 4H) |
| | 5,67-5,75 (m, 1H) |
| | 3,78-4,02 (m, 2H) |
| | 3,84 (s, 3H) |
| | 3,22 (s, 9H) |
| | 2,80-2,90 (m, 2H) |

## Beispiel 2

(R)-(-)-3-(2-Hydroxybenzoyloxy)-4-(trimethylammonio)-buttersäurebetain-Hydrobromid (Salicyloyl-L-carnitin-Hydrobromid)

In 200 ml einer 30%igen Lösung von Bromwasserstoff in Eisessig wurden 24,0 g o-Anisoyl-L-carnitin-Hydrochlorid (hergestellt gemäss Beispiel 1) gelöst und 6 h bei 60 °C gerührt. Anschliessend wurde das Reaktionsgemisch im Vakuum eingedampft und der Rückstand mit 200 ml Diethylether aufgeschlämmt und filtriert. Das kristalline Rohprodukt wurde zweimal aus je 110 ml heissem Isopropanol umkristallisiert.
Ausbeute: 14,0 g (50,2%) farblose Kristalle
Schmp.: 173-175 °C

$[\alpha]_D^{20}$ = -27,2° (c = 1, Wasser)

Beispiel 3

(R)-(-)-3-(2-Hydroxybenzoyloxy)-4-(trimethylammonio)-buttersäurebetain (Salicyloyl-L-carnitin)

In 130 ml Wasser wurden 13,0 g Salicyloyl-L-carnitin-Hydrobromid (hergestellt gemäss Beispiel 2) gelöst. Die Lösung wurde über eine Säule mit 58 g eines schwach basischen Anionenaustauscherharzes (Amberlite[R] IRA-93) filtriert und im Vakuum eingeengt. Durch Zugabe von 60 ml Aceton wurde das Produkt ausgefällt. Das kristalline Produkt wurde abfiltriert und bei 40°C im Vakuum getrocknet.
Ausbeute: 10,0 g (quantitativ) farblose Kristalle
Schmp.: 120-122°C
$[20]_D^{20}$ = -25,0° (c = 1, Wasser)

| | | | | | | |
|---|---|---|---|---|---|---|
| Elementaranalyse: | ber.<br>gef. | C 59,5<br>C 59,8 | H 6,9<br>H 6,8 | N 4,9<br>N 5,0 | Br -<br>Br <0,1 | Cl -<br>Cl <0,1 |

Magenverträglichkeitstest an Ratten (Ulcer Index)

Das R-(-)-3-(2-Hydroxybenzoyloxy)-4-(trimethylammonio)-buttersäurebetain (Salicoyl-L-Carnitin = SC) wurde im Vergleich Zu Acetylsalicylsäure (ASA) an männlichen Ratten getestet, indem anlehnend an die Methode von Okabe et al., Japan. J. Pharamcol. 1974, 24, 363ff, Magenschleimhautveränderungen induziert wurden.
Die Testsubstanzen wurden in einer 1% Carboxymethylcellulosesuspension (1% CMC) den Testratten p.o. verabreicht.
Die Magenschleimhautveränderungen wurden mittels dem Ulcer Index gemäß M.Chaumontet et al., Arzneimittelforschung, 1978, 28(II), Heft 11, 2119 - 2121 gemessen.
Tabelle 1 gibt die Ergebnisse wieder.

Tabelle 1

| Substanz | | Ulcer Index (U.I.) | Anzahl Ratten |
|---|---|---|---|
| Vergl. | 1% CMC 1 ml/250 g | 63,00 | 10 |
| Vergl. | ASA 200 $mg \cdot kg^{-1}$ | 300,00 | 20 |
| Erfi. | SC 200 $mg \cdot kg^{-1}$ | 144,00 | 10 |
| Erfi. | SC 500 $mg \cdot kg^{-1}$ | 200,00 | 10 |
| Erfi. | SC 1000 $mg \cdot kg^{-1}$ | 200,00 | 10 |
| CMC = Carboxymethylcellulose<br>ASA = Acetylsalicylsäure<br>SC = Salicoyl-L-Carnitin<br>Vergl. = Vergleich<br>Erfi. = Erfindung | | | |

**Patentansprüche**

1. 3-(2-Hydroxybenzoyloxy)-4-(trimethylammonio)-buttersäurebetain

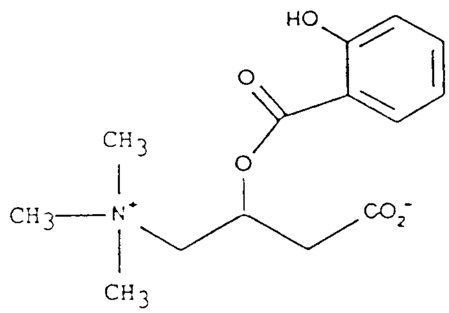

I

und dessen pharmazeutisch akzeptable Salze.

2. (R)-(-)-3-(2-Hydroxybenzoyloxy)-4-(trimethylammonio)-buttersäurebetain

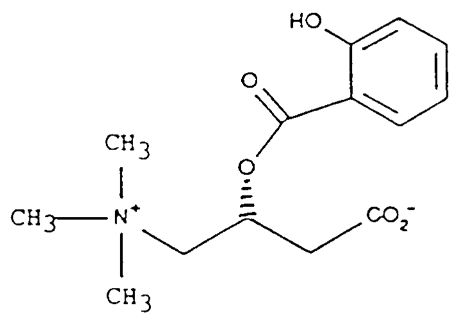

I I

und dessen pharmazeutisch akzeptable Salze als Verbindungen gemäss Patentanspruch 1.

3. (S)-( + )-3-(2-Hydroxybenzoyloxy)-4-(trimethylammonio)-buttersäurebetain

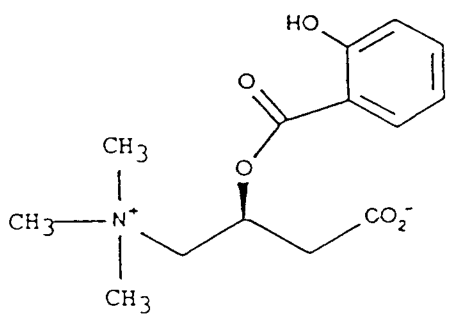

I I I

und dessen pharmazeutisch akzeptable Salze als Verbindungen gemäss Patentanspruch 1.

4. 3-(2-Hydroxybenzoyloxy)-4-(trimethylammonio)-buttersäure betain und dessen Salze zur Anwendung als therapeutische Wirkstoffe.

5. (R)-(-)-3-(2-Hydroxybenzoyloxy)-4-(trimethylammonio)-buttersäurebetain und dessen Salze zur Anwendung als therapeutische Wirkstoffe.

6. (S)-( + )-3-(2-Hydroxybenzoyloxy)-4-(trimethylammonio)-buttersäurebetainund dessen Salze zur Anwendung als therapeutische Wirkstoffe.

7. 3-(2-Hydroxybenzoyloxy)-4-(trimethylammonio)-buttersäurebetain und dessen Salze zur Anwendung als die Magenschleimhaut schonende Analgetika.

8. (R)-(-)-3-(2-Hydroxybenzoyloxy)-4-(trimethylammonio)-buttersäurebetain und dessen Salze zur Anwendung als die Magenschleimhaut schonende Analgetika.

9. (S)-(+)-3-(2-Hydroxybenzoyloxy)-4-(trimethylammonio)-buttersäurebetainund dessen Salze zur Anwendung als die Magenschleimhaut schonende Analgetika.

10. 3-(2-Methoxybenzoyloxy)-4-(trimethylammonio)-buttersäurebetain und dessen Salze.

11. (R)-(-)-3-(2-Methoxybenzoyloxy)-4-(trimethylammonio)-buttersäurebetain und dessen Salze.

12. (S)-(+)-3-(2-Methoxybenzoyloxy)-4-(trimethylammonio)-buttersäurebetainund dessen Salze.

13. Verfahren zur Herstellung von 3-(2-Hydroxybenzoyloxy)-4-(trimethylammonio)-buttersäurebetain, dadurch gekennzeichnet, dass ein 3-Hydroxy-4-(trimethylammonio)-buttersäurebetain-Hydrohalogenid in einer ersten Stufe mit einem 2-Methoxybenzoylhalogenid zu einem 3-(2-Methoxybenzoyloxy)-4-(trimethylammonio)-buttersäurebetain-Hydrohalogenid verestert wird, welches mit Bromwasserstoff in Essigsäure zu 3-(2-Hydroxybenzoyloxy)-4-(trimethylammonio)-buttersäurebetain-Hydrobromid demethyliert und mit einer Base zum Betain deprotoniert wird.

14. Verfahren nach Patentanspruch 13, dadurch gekennzeichnet, dass als 3-Hydroxy-4-(trimethylammonio)-buttersäurebetain-Hydrohalogenid das Hydrochlorid eingesetzt wird.

15. Verfahren nach Patentanspruch 13 oder 14, dadurch gekennzeichnet, dass als 2-Methoxybenzoylhalogenid das Säurechlorid eingesetzt wird.

16. Verfahren nach mindestens einem der Patentansprüche 13 bis 15, dadurch gekennzeichnet, dass als Base ein schwach basischer Anionenaustauscher eingesetzt wird.

17. Verfahren nach mindestens einem der Patentansprüche 13 bis 16, dadurch gekennzeichnet, dass als Ausgangsmaterial ein (R)-2-Hydroxy-4-(trimethylammonio)-buttersäurebetain-Hydrohalogenid (L-Carnitin-Hydrohalogenid) eingesetzt wird.

## Claims

1. 3-(2-Hydroxybenzoyloxy)-4-(trimethylammonio)-butyric acid-betaine

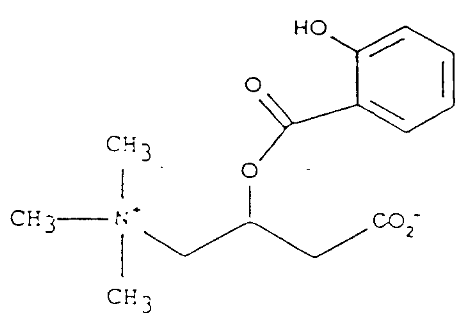

I

and pharmaceutically acceptable salts thereof.

2. (R)-(-)-3-(2-Hydroxybenzoyloxy)-4-(trimethylammonio)-butyric acid-betaine

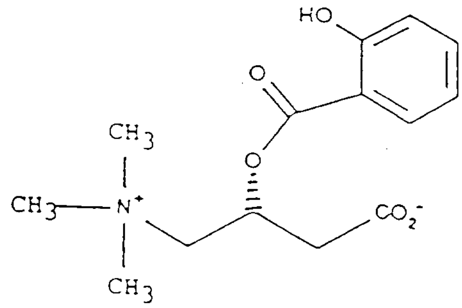

II

and pharmaceutically acceptable salts thereof as compounds according to claim 1.

3. (S)-(+)-3-(2-Hydroxybenzoyloxy)-4-(trimethylammonio)-butyric acid-betaine

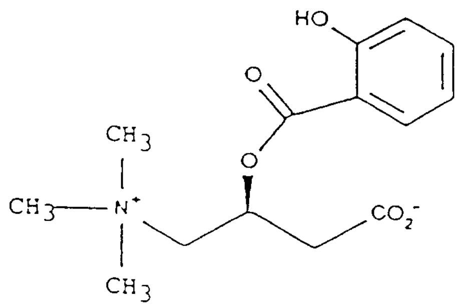

III

and pharmaceutically acceptable salts thereof as compounds according to claim 1.

4. 3-(2-Hydroxybenzoyloxy)-4-(trimethylammonio)-butyric acid-betaine and salts thereof for the use as therapeutically active substances.

5. (R)-(-)-3-(2-Hydroxybenzoyloxy)-4-(trimethylammonio)-butyric acid-betaine and salts thereof for the use as therapeutically active substances.

6. (S)-(+)-3-(2-Hydroxybenzoyloxy)-4-(trimethylammonio)-butyric acid-betaine and salts thereof for the use as therapeutically active substances.

7. 3-(2-Hydroxybenzoyloxy)-4-(trimethylammonio)-butyric acid-betaine and salts thereof for the use as analgetics which do not affect the stomach-lining.

8. (R)-(-)-3-(2-Hydroxybenzoyloxy)-4-trimethylammonio)-butyric acid-betaine and salts thereof for the use as analgetics which do not affect the stomach-lining.

9. (S)-(+)-3-(2-Hydroxybenzoyloxy)-4-(trimethylammonio)-butyric acid-betaine and salts thereof for the use as analgetics which do not affect the stomach-lining.

10. 3-(2-Methoxybenzoyloxy)-4-(trimethylammonio)-butyric acid-betaine and salts thereof.

11. (R)-(-)-3-(2-Methoxybenzoyloxy)-4-(trimethylammonio)-butyric acid-betaine and salts thereof.

12. (S)-(+)-3-(2-Methoxybenzoyloxy)-4-(trimethylammonio)-butyric acid-betaine and salts thereof.

13. Process for the preparation of 3-(2-hydroxybenzoyloxy)-4-(trimethylammonio)-butyric acid-betaine, characterised in that in a first stage a 3-hydroxy-4-(trimethylammonio)-butyric acid-betaine-hydrohalide is esterified with a 2-methoxybenzoyl halide to a 3-(2-methoxybenzoyloxy)-4-(trimethylammonio)-butyric acid-betaine-hydrohalide which latter is than demethylized with hydrogenbromide in acetic acid to give

3-(2-hydroxybenzoyloxy)-4-(trimethylammonio)-butyric acid-betaine-hydrobromide which is deprotonized with a base to give the respective betaine.

**14.** Process according to claim 13, characterised in that the hydrochloride is used as the 3-hydroxy-4-trimethylammonio)-butyric acid-betaine-hydrohalide.

**15.** Process according to claim 13 or 14, characterised in that the acid chloride is used as the 2-methoxybenzoyl halide.

**16.** Process according to at least one of claims 13 to 15, characterised in that a weakly basic anion exchanger is used as the base.

**17.** Process according to at least one of claims 13 to 16, characterised in that an (R)-2-hydroxy-4-(trimethylammonio)-butyric acid-betaine-hydrohalide (L-carnitine-hydrohalide) is used as the starting material.

**Revendications**

**1.** Bétaïne de l'acide 3-(2-hydroxybenzoyloxy)-4-(triméthylammonio)-butyrique

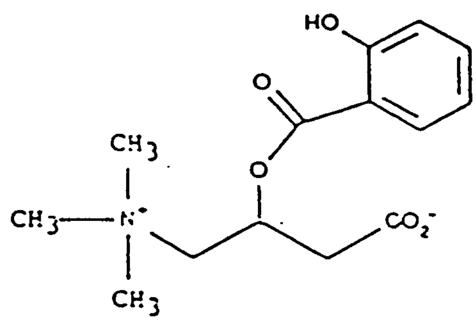

I

et ses sels pharmaceutiquement acceptables.

**2.** Bétaïne de l'acide (R)-(-)-3-(2-hydroxybenzoyloxy)-4-(triméthylammonio)-butyrique

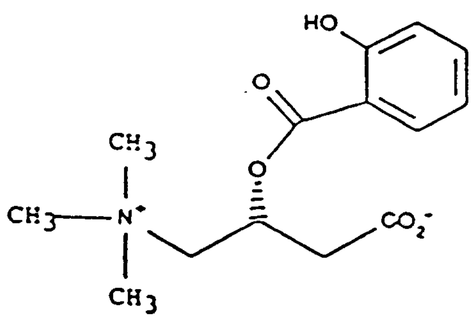

II

et ses sels pharmaceutiquement acceptables comme composés selon la revendication 1.

**3.** Bétaïne de l'acide (S)-( + )-3-(2-hydroxybenzoyloxy)-4-(triméthylammonio)-butyrique

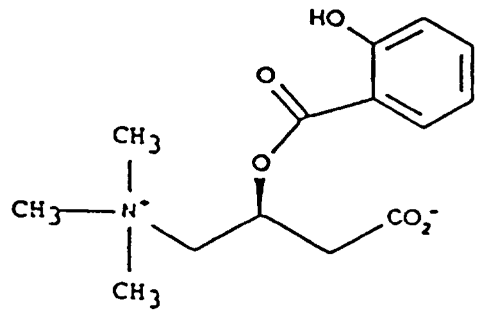

III

et ses sels pharmaceutiquement acceptables comme composés selon la revendication 1.

**4.** Bétaïne de l'acide 3-(2-hydroxybenzoyloxy)-4-(triméthylammonio)-butyrique et ses sels pour l'utilisation en tant que principes actifs thérapeutiques.

**5.** Bétaïne de l'acide (R)-(-)-3-(2-hydroxybenzoyloxy)-4-(triméthylammonio)-butyrique et ses sels pour l'application en tant que principes actifs thérapeutiques.

**6.** Bétaïne de l'acide (S)-( + )-3-(2-hydroxybenzoyloxy)-4-(triméthylammonio)-butyrique et ses sels pour l'application en tant que principes actifs thérapeutiques.

**7.** Bétaïne de l'acide 3-(2-hydroxybenzoyloxy)-4-(triméthylammonio)-butyrique et ses sels pour l'application en tant qu'analgésique ménageant les muqueuses stomacales.

**8.** Bétaïne de l'acide (R)-(-)-3-(2-hydroxybenzoyloxy)-4-(triméthylammonio)-butyrique et ses sels pour l'application en tant qu'analgésique ménageant les muqueuses stomacales.

**9.** Bétaïne de l'acide (S)-( + )-3-(2-hydroxybenzoyloxy)-4-(triméthylammonio)-butyrique et ses sels pour l'application en tant qu'analgésique ménageant les muqueuses stomacales.

**10.** Bétaïne de l'acide 3-(2-methoxybenzoyloxy)-4-(triméthylammonio)-butyrique et ses sels.

**11.** Bétaïne de l'acide (R)-(-)-3-(2-méthoxybenzoyloxy)-4-(triméthylammonio)-butyrique et ses sels.

**12.** Bétaïne de l'acide (S)-( + )-3-(2-méthoxybenzoyloxy)-4-(triméthylammonio)-butyrique et ses sels.

**13.** Procédé pour la préparation de bétaïne de l'acide 3-(2-hydroxybenzoyloxy)-4-(triméthylammonio)-butyrique, caractérisé en ce que l'on estérifie un hydrohalogénure de bétaïne de l'acide 3-hydroxy-4-(triméthylammonio)-butyrique dans une première étape avec un 2-méthoxybenzoylhalogénure en un hydrohalogénure-bétaïne de l'acide 3(2-méthoxybenzoyloxy)-4-(triméthylammonio)-butyrique, qui est déméthylé avec un bromure d'hydrogène dans l'acide acétique en bromhydrate-bétaïne de l'acide 3-(2-hydroxybenzoyloxy)-4-(triméthylammonio)-butyrique et déprotoner avec une base en bétaïne.

**14.** Procédé selon la revendication 13, caractérisé en ce qu'en tant qu'hydrohalogénure-bétaïne de l'acide 3-hydroxy-4-(triméthylammonio)-butyrique, on met en oeuvre le chlorhydrate.

**15.** Procédé selon la revendication 13 ou 14, caractérisé en ce qu'en tant que 2-méthoxybenzoylhalogénure, on met en oeuvre le chlorure d'acide.

**16.** Procédé selon au moins l'une des revendications 13 à 15, caractérisé en ce qu'en tant que base, on met en oeuvre un échangeur d'anion faiblement basique.

**17.** Procédé selon au moins l'une des revendications 13 à 16, caractérisé en ce qu'en tant que matière de départ, on met en oeuvre un hydrohalogénure-bétaïne de l'acide (R)-2-hydroxy-4-(triméthylammonio)-butyrique (L-carnitine-hydrohalogénure).